# EUROPEAN PATENT SPECIFICATION

(11) **EP 3 319 530 B1**
(45) Date of publication and mention of the grant of the patent: **29.01.2020**
(21) Application number: 16742124.7
(22) Date of filing: 09.07.2016
(51) Int. Cl.: A61B 17/12, A61B 90/00

(54) **VASCULAR OCCLUSION DEVICES**
GEFÄSSVERSCHLUSSVORRICHTUNGEN
DISPOSITIFS D'OCCLUSION VASCULAIRE

(30) Priority: 10.07.2015 US 201562191122 P
(43) Date of publication of application: 16.05.2018
(73) Proprietor: Boston Scientific Scimed, Inc., Maple Grove, MN 55311-1566 (US)
(72) Inventor: O'BRIEN, John-Allen, Co. Cork (IE); RYAN, Frank, Cork T12 FNC8 (IE); O'SULLIVAN, Conor, Cork (IE); KRUSCHKE, Eric, J., Cork (IE); FORDE, Declan, Cork (IE)
(74) Representative: Vossius & Partner Patentanwälte Rechtsanwälte mbB
(86) International application number: PCT/US2016/041665
(87) International publication number: WO 2017/011357

(56) References cited:
- EP-A2- 1 707 233
- WO-A1-2012/155093
- WO-A2-2009/124247
- US-A1- 2009 062 845
- US-A1- 2015 105 814

## Description

### FIELD OF THE DISCLOSURE

The present disclosure relates, *inter alia,* to devices, assemblies and kits for creating vascular occlusions and to methods for creating vascular occlusions using the same.

### BACKGROUND

The endovascular treatment of a variety of conditions throughout the body is an increasingly important form of therapy. Vascular occlusion devices are known which are placed within the vasculature of the body in order to form a physical barrier to blood flow and/or promote thrombus formation at the site. US2009/062845 A1 discloses a vascular occlusion device according to the preamble of claim 1.

The present disclosure pertains to improved devices, assemblies, kits and methods for blood vessel occlusion.

### SUMMARY

The vascular occlusion device according to the invention is defined in claim 1. Preferred embodiments of the invention are recited in the dependent claims. In some aspects, the present disclosure pertains to vascular occlusion devices.

The vascular occlusion devices have a proximal end and a distal end and comprise the following: (a) a support frame comprising a longitudinal axis and a longitudinal center, the support frame being self-expandable from a constrained shape to an unconstrained shape and comprising (i) a hub, (ii) a plurality of radial wire segments extending radially from the hub and (iii) a plurality of longitudinal wire segments extending longitudinally along the device and (b) a substantially-two dimensional insert attached to the support frame and comprising (i) a first insert portion in a first partially closed three-dimensional form that comprises a first open end and a first closed end, wherein the first open end faces in a proximal direction and the first closed end is positioned proximate the longitudinal center and (ii) a second insert portion in a second partially closed three-dimensional form that comprises a second open end and a second closed end, wherein the second open end faces in a distal direction and the second closed end is positioned proximate the longitudinal center.

In some embodiments of the preceding aspects, the first closed end of the insert portion may be attached to the second closed end of the second insert portion.

In some embodiments, which may be used in conjunction with any of the preceding aspects and embodiments, the first partially closed three-dimensional form and second partially closed three-dimensional form may be selected from a hollow conical form, a hollow pyramidal form and a hollow partial spheroidal form.

In some embodiments, which may be used in conjunction with any of the preceding aspects and embodiments, the insert may be a porous insert

In some embodiments, which may be used in conjunction with any of the preceding aspects and embodiments, the insert may comprise a polymeric material.

In some embodiments, which may be used in conjunction with any of the preceding aspects and embodiments, the support frame may comprise from four to eight radial wire segments and from four to eight longitudinal segments.

In some embodiments, which may be used in conjunction with any of the preceding aspects and embodiments, the radial wire segments and the longitudinal wire segments may be linear or substantially linear.

In some embodiments, which may be used in conjunction with any of the preceding aspects and embodiments, the radial wire segments may comprise a first radial wire segment end and a second radial wire segment end, wherein the first radial wire segment end is attached to the hub, and wherein the second radial wire segment end is attached to one of the longitudinal wire segments.

In some embodiments, a vascular occlusion device in accordance with any of the preceding aspects and embodiments may further comprise an additional hub and a plurality of additional radial wire segments extending radially from the additional hub. In certain of these embodiments, the additional radial wire segments may comprise a first additional radial wire segment end and a second additional radial wire segment end, wherein the first additional radial wire segment end is attached to the additional hub, and wherein the second additional radial wire segment end is attached to one of the longitudinal wire segments.

In some embodiments, which may be used in conjunction with any of the preceding aspects and embodiments, the hub may comprise an attachment feature.

In some embodiments, which may be used in conjunction with any of the preceding aspects and embodiments, the support frame may comprise a plurality of anchors.

Other aspects of the present disclosure pertain to assemblies that comprise a vascular occlusion device in accordance with any of the preceding aspects and embodiments and an elongated delivery member that is configured to be attached to and detached from the vessel occlusion device.

Other aspects of the present disclosure pertain to kits which comprise a vascular occlusion device in accordance with any of the preceding aspects and embodiments, a tubular delivery device and, optionally, an elongated delivery member that is configured to be attached to and detached from the vascular occlusion device. In some instances, the vascular occlusion device may be compressed and preloaded into the tubular device in the constrained shape.

Examples of attachment configurations for any of the preceding assemblies and kits include the following, among others: (a) the elongated delivery member and the vascular occlusion device may comprise interlocking arms, (b) the elongated delivery member and the vascular occlusion device may comprise a threaded male member and a threaded female receptacle, or (c) the elongated delivery member and the vascular occlusion device may be configured to be detached by electrolysis.

Other aspects of the present disclosure not forming part of the invention pertain to methods in which (a) a vascular occlusion device in accordance with any of the preceding aspects and embodiments is introduced into a blood vessel while in a constrained shape and (b) removing a constraint that maintains the vascular occlusion device in the constrained shape, such that the support frame self-expands and the device contacts a wall of the blood vessel and the insert impedes flow through the blood vessel. In certain embodiments, the constraint is removed by ejecting the vascular occlusion device from a tubular medical device.

Various additional aspects, embodiments, and benefits of the present disclosure will become immediately apparent to those of ordinary skill in the art upon review of the detailed description and claims to follow.

### BRIEF DESCRIPTION OF THE DRAWINGS

Non-limiting embodiments of the present disclosure are described by way of example with reference to the accompanying figures, which are schematic and not intended to necessarily be drawn to scale. In the figures, each identical or nearly identical component illustrated is typically represented by a single numeral. For purposes of clarity, not every component is labeled in every figure, nor is every component of each embodiment of the disclosure shown where illustration is not necessary to allow those of ordinary skill in the art to understand the disclosure. In the figures:
Fig. 1A is a schematic side view of a self-expanding occlusion device, in accordance with an embodiment of the present disclosure;
Fig. 1B is a is a schematic isometric view of the occlusion device of Fig. 1A;
Fig. 1C is a is a schematic side view of the occlusion device of Fig. 1A; and
Fig. 2A, Fig. 2B and Fig. 2C are schematic partial cross-sectional views illustrating three points of time during a procedure in which an occlusion device in accordance with an embodiment of the present disclosure is deployed in the vasculature.

### DETAILED DESCRIPTION

A more complete understanding of the present disclosure is available by reference to the following detailed description of numerous aspects and embodiments of the disclosure. The detailed description which follows is intended to illustrate but not limit the disclosure.

The terms "proximal" and "distal" generally refer to the relative position, orientation, or direction of an element or action, from the perspective of a clinician using the medical device, relative to one another. Thus, "proximal" may generally be considered closer to the clinician or an exterior of a patient, and "distal" may generally be considered to be farther away from the clinician, along the length or beyond the end of the medical device.

The present disclosure pertains to devices, assemblies and kits for creating blood vessel occlusions. The occlusion devices of the present disclosure are collapsible to fit within a tubular device such as a catheter or delivery sheath and, when removed from the tubular device, can naturally expand toward an unconstrained configuration to fully occlude a blood vessel. For example, in certain embodiments the occlusion devices may be pushed through and/or from the distal end of a catheter (e.g., ranging from 0.021" (0.53 mm) microcatheter to a 5 French (1.67 mm) guide catheter) that is in place at the site of embolization, among others. Upon exiting the catheter, the device will automatically expand to the diameter of the blood vessel, occluding the same.

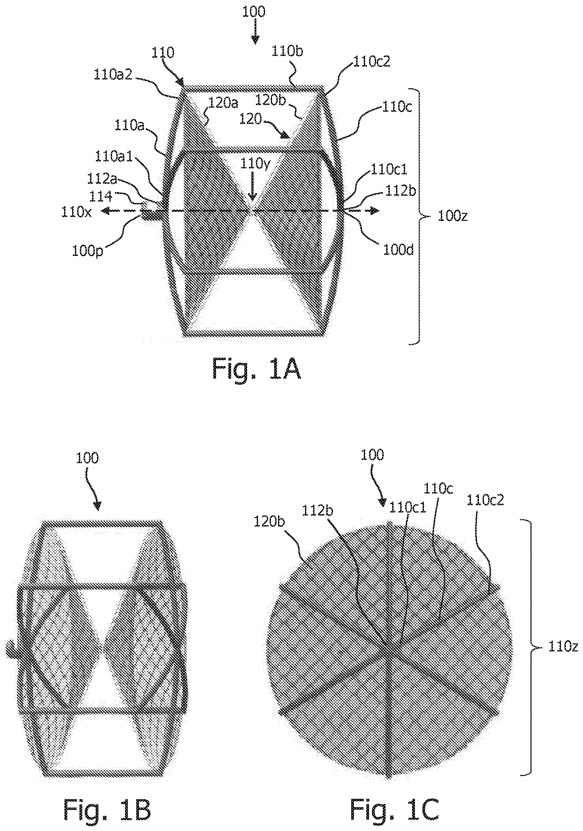

FIG. 1A is a schematic side illustration of a vascular occlusion device 100 in an unconstrained state, in accordance with an embodiment of the present disclosure. Fig. 1B is an isometric view of the occlusion device of Fig. 1A, and Fig. 1C is a side view of the occlusion device 100 of Fig. 1A. The vascular occlusion device 100 shown has a proximal end lOOp and a distal end 100d. The vascular occlusion device 100 also comprises a self-expanding support frame 110 having a longitudinal axis 110x, a longitudinal center 110y and a diameter 110z. The support frame 110 may comprise a plurality of wire segments including a plurality of radial wire segments 110a extending radially from the axis 110x, in particular, extending radially from a hub 112a in the embodiment shown. The support frame 110 may also comprise a plurality of longitudinal wire segments 110b extending longitudinally relative to the longitudinal axis 110x, in particular, parallel to the longitudinal axis 110x in the embodiment shown. Each radial wire segment 110a in the embodiment shown has a first radial wire segment end portion 110a1 and a second radial wire segment end portion 110a2, wherein the first radial wire segment end portion 110a1 is attached to the hub 112a and the second radial wire segment end portion 110a2 is attached to one of the longitudinal wire segments 110b.

The hub 112a may be integrally formed with the radial wire segments 110a or may be a separate component from the radial wire segments 110a, for example, one that is attached to the radial wire segments 110a, which may be, for example, soldered, welded, fused, glued, crimped, or otherwise joined together to the radial wire segments 110a.

The support frame 110 shown further includes a delivery feature 114 for attachment to and detachment from an elongate delivery member. Strategies for reversible attachment include mechanical (e.g. threads, clamps, interlocking arms, etc.) and electrical (e.g., electrolytic dissolution, etc.) strategies. Some particular examples of delivery features include an arm (e.g., an atraumatic linking arm as shown) suitable for interlocking with a complementary arm on a delivery device, a male or female threaded feature for engagement with a complementary female or male threaded feature on a delivery device, and so forth. While the delivery feature 114 shown is of a unitary structure with the support frame 110, delivery feature 114 may also be provided in the form of a separate component that is attached to the support frame 110, for example, soldered, welded, fused, glued, crimped, or otherwise joined to the support frame 110. In another embodiment, for example, where the occlusion device 100 is merely pushed from a catheter or sheath, no such delivery feature 114 is present.

The support frame 110 may optionally further comprise a plurality of additional radial wire segments 110c extending radially from an additional hub 112b. Each additional radial wire segment 110c in the embodiment shown has a first additional radial wire segment end portion 110c1 and second additional radial wire segment end portion 110c2, wherein the first additional radial wire segment end portion 110c1 is attached to the additional hub 112b and the second additional radial wire segment end portion 110c2 is attached to one of the longitudinal wire segments 110b.

The additional hub 112b may be integrally formed with the additional radial wire segments 110c (in the embodiment shown, the additional hub 112b is simply an intersection point where the additional radial wire segments 110c meet), or it may be a separate component from the additional radial wire segments 110c that is attached to the additional radial wire segments 110c, for example, soldered, welded, fused, glued, crimped, or otherwise joined together to the additional radial wire segments 110c.

In various embodiments, the point where each first radial wire segment end portion 110a1 is attached to the hub 112a is proximal to the point where the second radial wire segment end portion 110a2 is attached to one of the longitudinal wire segments 110b. In the embodiment shown, this is achieved by using curved (specifically, arc-shaped) radial wire segments 110a, but could also be achieved by various other approaches including angling the radial wire segments 110a distally from the hub 112a.

Analogously, in various embodiments, the point where each first additional radial wire segment end portion 110c1 is attached to the additional hub 112b is distal to the point where the second additional radial wire segment end 110c2 is attached to one of the longitudinal wire segments 110b. As above, in the embodiment shown, this is achieved by using curved (specifically, arc-shaped) additional radial wire segments 110c, but could also be achieved by various other approaches including angling the additional radial wire segments 110c proximally from the additional hub 112b.

In various embodiments, the radial wire segments 110a, additional radial wire segments 110c and longitudinal wire segments 110b are linear or substantially linear, for example, comprising an arc having a radius of curvature of less than 20 degrees or comprising no angle less than 160 degrees (wherein 180 degrees is linear).

In the embodiment shown, there are six radial wire segments 110a, six longitudinal wire segments 110b, and six additional radial wire segments 110c. However, other numbers of these segments may be employed including 3, 4, 5, 7, 8, 9, 10 or more. Moreover, while the radial wire segments 110a, longitudinal wire segments 110b, and additional radial wire segments 110c are evenly spaced in rotation around the longitudinal axis 110x in the embodiment shown, this is not required.

The vascular occlusion device 100 also comprises a flexible substantially two-dimensional insert 120 disposed within and attached to the support frame 110. As used herein, a "substantially two-dimensional" object is one where the thickness of the object is much less that the other dimensions (e.g., diameter, length/width, etc.), typically having a thickness that 10% or less, 5% or less, or even 1% or less, relative to the other dimensions.

The insert 120 assists the device in slowing or immediately halting blood flow upon expansion of the support frame 110 in a blood vessel. Thus, in various embodiments, the support frame 110 acts to anchors the device to the vessel, whereas the insert is designed to reduce or eliminate blood flow.

In the embodiment shown, the insert 120 comprises a first insert portion 120a in the form of a first partially closed three-dimensional form having an open end facing in a proximal direction and a closed end proximate the longitudinal center 110y of the support frame 110, wherein the diameter of the first insert portion 120a increases in a distal-to-proximal portion. The insert 120 also comprises a second insert portion 120b in the form of a second partially closed three-dimensional form having an open end facing in a distal direction and a closed end proximate the longitudinal center 110y of the support frame 110, wherein the diameter of the second insert portion 120b increases in a proximal-to-distal direction.

In the particular embodiment shown, the first insert portion 120a is attached to the second insert portion 120b, although this is not required.

In the particular embodiment shown, the first insert portion 120a and second insert portion 120b each comprise a hollow conical structure. Other structures, however, are possible including hollow pyramidal structures and hollow partial spheroidal structures, among others. Examples of hollow pyramidal structures include hollow structures based on pyramids having a base with 3, 4, 5, 6, 7, 8, 9, 10 or more sides. Examples of hollow partial spheroidal structures include hollow spheroidal caps (formed by a plane passing through a spheroid such as a sphere, oblate spheroid, or prolate spheroid) including spherical caps such as hemispheres, among others.

Because the first insert portion 120a faces in a proximal direction and the second insert portion 120b faces in a distal direction, the device is configured to effectively block fluid flow in both a proximal direction and a distal direction.

In some embodiments, the insert 120 may be permeable to blood and/or other fluids, such as water. In some embodiments, the insert 120 may be impermeable to such fluids.

In some embodiments, an insert 120 may be selected that promotes endothelialization after implantation.

In certain embodiments, the vascular occlusion device 100 may have an expanded diameter that is 10-30% greater than the diameter of a vessel to be embolized such that radial force assists in anchoring the device 100. Anchoring may also be assisted by pressure associated with the direction of blood flow in embodiments where the vessel to be embolized narrows in the direction of blood flow (e.g., arterial embolization).

In certain embodiments, at least a portion of the outer tissue-engaging surfaces of the wire segments of the support frame 110 (e.g., outer surfaces of each longitudinal wire segment 110b) may be roughened to better engage surrounding tissue. Alternatively or in addition, in certain embodiments the occlusion device may include a plurality of anchors (e.g., barbs, hooks, etc.) extending radially outward from the support frame such that they can engage tissue and inhibit longitudinal movement of the deployed vascular occlusion device. For instance, the support frame may be provided with a plurality of hooks or barbs around its circumference (e.g., with one or more hooks or barbs on each longitudinal wire segment 110b).

Because the geometry of the occlusion devices described herein is readily scalable, occlusion devices can be manufactured in a variety of different outer diameters and lengths.

For example, a vascular occlusion device having a compressed diameter sufficiently small to occupy a 0.021 inch inner diameter catheter (i.e., less than 0.021 inch or 0.53 mm) may have an unconstrained diameter ranging from 3 mm to 22 mm. Such devices are, for example, suitable for embolization of a vessel having an inner diameter ranging from 2 mm to 18 mm.

Such devices are, for example, suitable for embolization of a vessel having an unrestrained length ranging from 6 mm to 18 mm. The device when deployed may have a length 10-40% greater than unrestrained length.

In various embodiments, the support frame may be formed of a material that has a shape memory that allows the material to be compressed and constrained at a reduced compressed diameter and subsequently expand from the compressed diameter upon removal of the constraint. Such material may be selected, for example, from a metallic material, a metallic alloy, a ceramic material, a polymer, a metallic-polymer composite, a ceramic-polymer composite, combinations thereof, and the like. Particularly beneficial materials include metallic materials and/or alloys such as nickel-titanium alloy (nitinol) (e.g., super elastic or linear elastic nitinol), stainless steel (e.g., 303, 304v, or 316L stainless steel), nickel-chromium alloy, nickel-chromium-iron alloy, cobalt alloy, nickel, titanium, platinum, and the like. In various embodiments, the hub may be formed from such materials as well.

Support frames may be formed from these and other materials by various methods. For example, in certain embodiments, a support frame may be formed from a length of one or more wires wound onto a shaping mandrel and heated to impart shape memory to the wire. One particularly beneficial wire is nitinol, which is desirable, for example, due to its fatigue resistant properties and its ability to be compressed inside a delivery system with excellent shape recovery. Once wound onto the mandrel, the nitinol wire may be heat-treated above its transition temperature to impart memory into the wire. The wire will assume the shape of the shaping mandrel. In some embodiments, a support frame may be cut from a tubular member, such as a metallic hypotube, or other suitable starting substrate. In some embodiments, the support frame may be formed in a mold from a melted material.

In some embodiments, a hub may be integrally formed with the support frame. In some embodiments, after forming the support frame, a plurality of free ends of the radial segments of the support frame may be fixedly attached to a hub, for example, by adhesive(s), welding or soldering, friction fit, or other mechanical means. In some embodiments, the hub is provided with an engagement feature such as a linking arm or a male or female threaded member, among other possibilities.

Beneficial materials for the inserts described herein include polyesters such as polyethylene terephthalate (PET), polyamides such as nylon, polyurethanes, fluoropolymers such as polytetrafluoroethylene (PTFE), including expanded polytetrafluoroethylene (ePTFE), and polyvinylidene difluoride (PVDF), polyalkylenes such as polyethylene, or other suitable biostable materials known in the art. Temporary covering materials may also be used in some embodiments and include polyesters such as polylactide, polyglycolide, poly(lactide-co-glycolide), or other suitable biodegradable materials known in the art, and natural tissue such as human tissue and decellularized plant and animal tissue.

The insert may be porous or non-porous. Porous inserts may be formed by a number of techniques including fiber-based techniques and techniques where a membrane is rendered porous. Fiber-based fabrication techniques include, for example, various woven and non-woven techniques, including weaving, knitting, braiding, electrospinning, electrospraying, fusion techniques where fibers are fused to one another (e.g., felt-forming techniques), among others. Techniques where a membrane is rendered porous include laser cutting, molding and stretching (e.g., ePTFE).

Pores, where present, may vary widely in diameter. In certain embodiments, pore size may be less than the size of a normal platelet (6-8 µm), for example ranging from 50 nm to 5 µm, in diameter.

In some embodiments a flat sheet of material (e.g., a porous or non-porous polymeric material) may be formed into a partially closed three-dimensional form, for example, by molding in the presence of heat and/or pressure or by combining multiple pieces of material (e.g., where a pyramidal form is formed, with a separate piece of material for each face), among other techniques.

In other embodiments, a partially closed three-dimensional form is formed without a flat material intermediate, for example, by directly forming a woven or non-woven fibrous material in the shape of a partially closed three-dimensional form (e.g., by spinning, weaving, knitting, or otherwise consolidating one or more fibers in the desired partially closed three-dimensional form), by injection molding a polymer into the desired form partially closed three-dimensional form, and so forth.

In some embodiments, the insert is formed on support frame (e.g., by weaving the insert directly on the support frame).

In some embodiments, a preformed insert is threaded onto the support frame (e.g., an insert may be threaded onto a frame comprising a first hub, radial segments and longitudinal segments and the support frame subsequently completed by the attachment of an additional hub and additional radial segments).

In some embodiments, a preformed insert may be attached to the expandable frame by various methods. For example, in some embodiments, the support frame may include a plurality of barbs or other anchors, which may project through the insert, holding it in place. In some embodiments, the insert may be attached to the support frame by other suitable attachment means, such as adhesives, threads including sutures, staples, welding or soldering, or other suitable attachment strategy.

In some embodiments the insert is wrapped around a portion of the support frame and attached to itself, or a first sheet of insert material is attached to a second sheet of insert material capturing portions of the frame the sheets, using a suitable mechanism such as heat bonding, pressure bonding, solvent bonding, adhesive bonding, threads including sutures, staples, or other suitable attachment strategy.

In various embodiments, the occlusion device may include imaging markers, for example, radiopaque markers, which may be positioned at one or more points along the device including (a) the distal end and/or proximal end and/or (b) certain points around the circumference and/or along the length of the support frame. The hub and/or detachment feature may also be formed from or provided with a radiopaque material. In a particularly beneficial embodiment, radiopaque markers may be provided at opposing ends of the device. Radiopaque markers may be, for example, attached, electroplated, dipped and/or coated at one or more locations on the device. Radiopaque materials are understood to be materials capable of producing a relatively bright image on a fluoroscopy screen or another imaging technique such as X-ray during a medical procedure. This relatively bright image aids the user of the device in determining its position and/or orientation. Suitable radiopaque materials may include, but are not limited to metals such as gold, platinum, palladium, tantalum, tungsten, metal alloys comprising one or more of the preceding metals, bismuth subcarbonate, iodine and the like.

In some embodiments, the vascular occlusion device may be coated with, or may otherwise include a material that provides a smooth, slick outer surface. In some embodiments, the vascular occlusion device may include or be coated with a lubricious coating, a hydrophilic coating, a hydrophobic coating, a drug-eluting material, or other suitable coating depending on the intended use or application.

In some embodiments, vascular occlusion devices are provided in conjunction with a delivery system that includes an elongate delivery member and tubular delivery device.

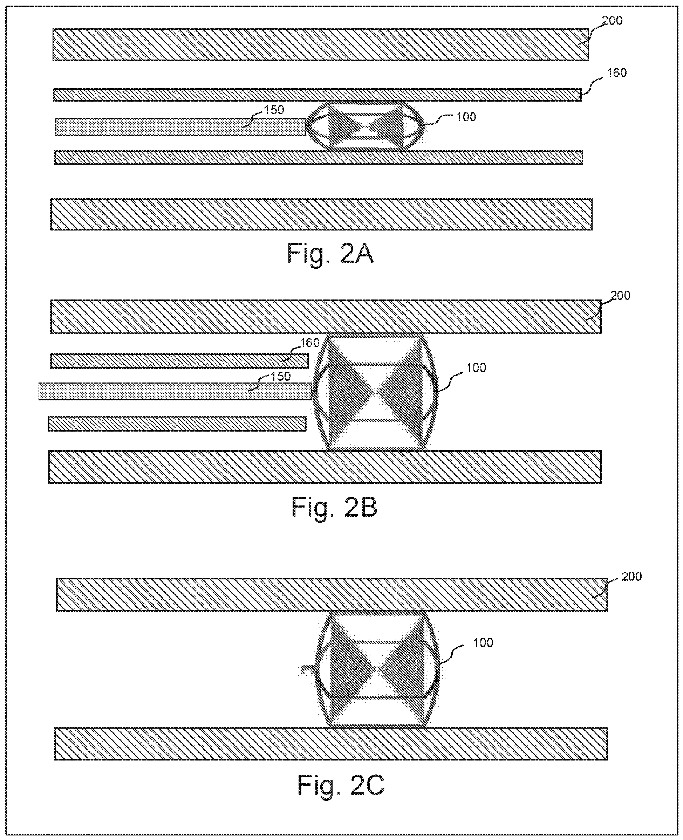

With reference to Figs. 2A and 2B, for delivery, the vascular occlusion device 100 may be compressed within a lumen of a tubular device such as a delivery catheter 160, in a constrained position. An elongate delivery member such as a push rod or delivery shaft 150 may also be disposed within the lumen of the delivery catheter 160 and, in some embodiments, may be reversibly connected to the vascular occlusion device 100 at the proximal end, such that the vascular occlusion device 100 can be advanced and withdrawn relative to the catheter 160 as desired and eventually released within the body. For example the delivery shaft 150 may comprise an arm that interlocks with a complementary arm attached to the device, among various other possible mechanical (e.g. male and female threaded components, etc.) and electrical (e.g., electrolytic dissolution, etc.) ways of forming such a reversible connection. The delivery catheter 160, vascular occlusion device 100 and delivery shaft 150 collectively form a delivery system.

The delivery system may be percutaneously introduced into a patient to deliver the vascular occlusion device 100 to a desired vascular site 200 (e.g., an artery, vein, etc.). Access to an artery or vein to be embolized may be achieved via the femoral artery, femoral vein, or radial artery, among other access points. Initially, the vascular occlusion device 100 may be disposed in a first, constrained position within the lumen of the delivery catheter 160, as shown in Fig. 2A.

Upon reaching the desired delivery location, the delivery catheter 160 may be withdrawn proximally while keeping the delivery shaft stationary, or the delivery shaft 150 may be advanced distally while the delivery catheter 160 is held stationary (i.e., relative movement between the delivery catheter 160 and the delivery shaft 150 occurs), such that the vascular occlusion device 100 emerges from the delivery catheter 160 and self-expands radially outward to an expanded position such that the outer surface of the vascular occlusion device 100 conforms to the wall of the vascular site 200 as shown in Fig. 2B. Lastly, if attached, the delivery shaft 150 may be disconnected from the proximal hub 112 and the delivery catheter 160 and delivery shaft 150 removed from the patient, leaving the vascular occlusion device at the vascular site 200 as shown in Fig. 2C.

Once implanted, the insert acts to slow or halt blood flow. In some embodiments, the device may act as a substrate for coagulation, creating a permanent embolus. In certain beneficial embodiments, the device may become integrated to the vascular tissue. In some embodiments, the insert material physically blocks blood flow (e.g., where the insert material is non-porous or has very small pores), without relying on a coagulation cascade to form the blockage (*cf*., embolic coils and other devices which require a coagulation cascade to form a blockage). This is advantageous, for instance, in patients with coagulopathies (e.g. patients having dysfunctional clotting cascades such that clots do not readily form).

Using these and other procedures, the blood vessel embolization devices described herein may be implanted into a wide variety of vessels to be embolized, including a wide variety of arterial and venous blood vessels. Examples of arteries in which the blood vessel embolization devices may be implanted include the following arteries (including any divisions thereof): internal iliac artery (hypogastric artery), external iliac artery, gastroduodenal artery, renal artery, hepatic artery, uterine artery, lienal artery, splenic artery, intercostals artery, mesenteric artery, right gastric artery, left gastric artery, lumbar artery, internal carotid artery, communicating artery, basilar artery, bronchial artery, cerebral artery, cerebellar artery, profunda femoris artery, gastroepiploic artery, and pancreaticoduodenal artery, among others. Examples of veins in which the blood vessel embolization devices may be implanted include a pelvic vein, internal iliac vein (hypogastric vein), portal vein and gonadal veins (e.g. spermatic vein or ovarian vein, depending on gender), among others. Examples of blood vessels in which the blood vessel embolization devices may be implanted further include abnormal blood vessels, for example, arteriovenous fistulas and arteriovenous malformations, among others.

In particularly beneficial embodiments, blood vessel embolization devices as described herein may be employed to perform prophylactic gastroduodenal artery embolization (the gastroduodenal artery is a branch of the common hepatic artery) and/or right gastric artery embolization, for example, prior to Y90 therapy or other microsphere therapy for hepatocellular carcinoma or liver metastases (e.g., drug eluting microspheres, TACE, etc.) as well as to perform prophylactic hypogastric embolization (the hypogastric artery is also known as the internal iliac artery) prior to AAA stent graft implantation.

The vascular occlusion devices described herein are advantageous in these and other procedures in that they are configured to block blood flow with only a single deployment and in that vessels can be embolized in which the direction of blood flow is either proximal or distal.

In another aspect of the disclosure, medical kits useful in embolization procedures are provided. The medical kits may include all or a subset of all the components useful for performing the procedures. For example, the medical kits may comprise any combination of any two, three, four, or more of the following items: (a) a vessel occlusion device as described herein, (b) a tubular device (e.g., a catheter or sheath) suitable for delivering the vessel occlusion device (in certain beneficial embodiments, the vessel occlusion device may be compressed and preloaded into the tubular device in a constrained, i.e., reduced diameter, shape), (c) an elongate delivery member such as a delivery shaft, which may be reversibly connected to the vascular occlusion device via a suitable mechanism such as one of those described hereinabove, (d) a catheter introducer, (e) suitable packaging material, and (f) printed material with one or more of the following: storage information and instructions regarding how to deploy the vessel occlusion device in a subject.

Although various embodiments are specifically illustrated and described herein, it will be appreciated that modifications and variations of the present disclosure are covered by the above teachings and are within the purview of the appended claims.

## Claims

1. A vascular occlusion device (100) having a proximal end (100p) and a distal end (100d), said occlusion device comprising: (a) a support frame (110) comprising a longitudinal axis (110x) and a longitudinal center (110y), the support frame being self-expandable from a constrained shape to an unconstrained shape and comprising (i) a hub (112a), (ii) a plurality of radial wire segments (110a) extending radially from the hub and (iii) a plurality of longitudinal wire segments (110b) extending longitudinally along the device and (b) a substantially sheet-like insert (120) attached to said support frame charaterised in that the substantially sheet-like insert (120) comprises (i) a first insert portion (120a) in a first partially closed three-dimensional form that comprises a first open end and a first closed end, wherein the first open end faces in a proximal direction and the first closed end is positioned proximate the longitudinal center and (ii) a second insert portion (120b) in a second partially closed three-dimensional form that comprises a second open end and a second closed end, wherein the second open end faces in a distal direction and the second closed end is positioned proximate the longitudinal center (110y).

2. The vascular occlusion device of claim 1, wherein the first closed end of the insert portion is attached to the second closed end of the second insert portion.

3. The vascular occlusion device of any of claims 1-2, wherein the first partially closed three-dimensional form and a second partially closed three-dimensional form are selected from a hollow conical form, a hollow pyramidal form and a hollow partial spheroidal form.

4. The vascular occlusion device of any of claims 1-3, wherein the insert is a porous insert, wherein the insert which comprises a polymeric material, or both.

5. The vascular occlusion device of any of claims 1-4, wherein the support frame comprises from four to eight radial wire segments and from four to eight longitudinal segments.

6. The vascular occlusion device of any of claims 1-5, wherein the radial wire segments and the longitudinal wire segments are linear or substantially linear.

7. The vascular occlusion device of any of claims 1-6, wherein said radial wire segments comprise a first radial wire segment end and a second radial wire segment end, wherein the first radial wire segment end is attached to the hub, and wherein the second radial wire segment end is attached to one of said longitudinal wire segments.

8. The vascular occlusion device of any of claims 1-7, further comprising an additional hub and a plurality of additional radial wire segments extending radially from the additional hub.

9. The vascular occlusion device of claim 8, wherein the additional radial wire segments comprise a first additional radial wire segment end and a second additional radial wire segment end, wherein the first additional radial wire segment end is attached to the additional hub, and wherein the second additional radial wire segment end is attached to one of said longitudinal wire segments.

10. The vascular occlusion device of any of claims 1-9, wherein the hub comprises an attachment feature.

11. The vascular occlusion device of any of claims 1-10, wherein the support frame comprises a plurality of anchors.

12. An assembly comprising the vascular occlusion device of any of claims 1-11 and an elongated delivery member that is configured to be attached to and detached from the vessel occlusion device.

13. The assembly of claim 12, wherein the elongated delivery member and the vascular occlusion device comprise interlocking arms, wherein the elongated delivery member and the vascular occlusion device comprise a threaded male member and a threaded female receptacle, or wherein the elongated delivery member and the vascular occlusion device are configured to be detached by electrolysis.

14. A kit comprising a vascular occlusion device in accordance with any of claims 1-11, a tubular delivery device and, optionally, an elongated delivery member that is configured to be attached to and detached from the vascular occlusion device.

15. The kit of claim 14, wherein the vascular occlusion device is compressed and preloaded into the tubular device in said constrained shape.

## Patentansprüche

1. Gefäßverschlussvorrichtung (100) mit einem proximalen Ende (100p) und einem distalen Ende (100d), wobei die Gefäßverschlussvorrichtung aufweist: (a) einen Stützrahmen (110), der eine Längsachse (110x) und einen Längsmittelpunkt (110y) aufweist, wobei der Stützrahmen von einer eingezwängten Form in eine uneingezwängte Form selbstexpandierbar ist und (i) eine Nabe (112a), (ii) mehrere Radialdrahtsegmente (110a), die sich radial von der Nabe aus erstrecken, und (iii) mehrere Längsdrahtsegmente (110b), die sich in Längsrichtung entlang der Vorrichtung erstrecken, aufweist, und (b) einen im Wesentlichen bahnartigen Einsatz (120), der an dem Stützrahmen befestigt ist,
**dadurch gekennzeichnet, dass** der im Wesentlichen bahnartige Einsatz (120) (i) ein erstes Einsatzteil (120a) in einer ersten teilweise geschlossenen dreidimensionalen Form, die ein erstes offenes Ende und ein erstes geschlossenes Ende aufweist, wobei das erste offene Ende in eine proximale Richtung weist und das erste geschlossene Ende nahe dem Längsmittelpunkt positioniert ist, und (ii) ein zweites Einsatzteil (120b) in einer zweiten teilweise geschlossenen dreidimensionalen Form aufweist, die ein zweites offenes Ende und ein zweites geschlossenes Ende aufweist, wobei das zweite offene Ende in eine distale Richtung weist und das zweite geschlossene Ende nahe dem Längsmittelpunkt (110y) positioniert ist.

2. Gefäßverschlussvorrichtung nach Anspruch 1, wobei das erste geschlossene Ende des Einsatzteils am zweiten geschlossenen Ende des zweiten Einsatzteils befestigt ist.

3. Gefäßverschlussvorrichtung nach einem der Ansprüche 1-2, wobei die erste teilweise geschlossene dreidimensionale Form und eine zweite teilweise geschlossene dreidimensionale Form aus einer Hohlkegelform, einer Hohlpyramidenform und einer hohlen Teilkugelform ausgewählt sind.

4. Gefäßverschlussvorrichtung nach einem der Ansprüche 1-3, wobei der Einsatz ein poröser Einsatz ist und/oder der Einsatz ein polymeres Material aufweist.

5. Gefäßverschlussvorrichtung nach einem der Ansprüche 1-4, wobei der Stützrahmen vier bis acht Radialdrahtsegmente und vier bis acht Längsdrahtsegmente aufweist.

6. Gefäßverschlussvorrichtung nach einem der Ansprüche 1-5, wobei die Radialdrahtsegmente und die Längsdrahtsegmente linear oder im Wesentlichen linear sind.

7. Gefäßverschlussvorrichtung nach einem der Ansprüche 1-6, wobei die Radialdrahtsegmente ein erstes Radialdrahtsegmentende und ein zweites Radialdrahtsegmentende aufweisen, wobei das erste Radialdrahtsegmentende an der Nabe befestigt ist und wobei das zweite Radialdrahtsegmentende an einem der Längsdrahtsegmente befestigt ist.

8. Gefäßverschlussvorrichtung nach einem der Ansprüche 1-7, ferner aufweisend eine zusätzliche Nabe und mehrere zusätzliche Radialdrahtsegmente, die sich radial von der zusätzlichen Nabe aus erstrecken.

9. Gefäßverschlussvorrichtung nach Anspruch 8, wobei die zusätzlichen Radialdrahtsegmente ein erstes zusätzliches Radialdrahtsegmentende und ein zweites zusätzliches Radialdrahtsegmentende aufweisen, wobei das erste zusätzliche Radialdrahtsegmentende an der zusätzlichen Nabe befestigt ist und wobei das zweite zusätzliche Radialdrahtsegmentende an einem der Längsdrahtsegmente befestigt ist.

10. Gefäßverschlussvorrichtung nach einem der Ansprüche 1-9, wobei die Nabe eine Befestigungseinrichtung aufweist.

11. Gefäßverschlussvorrichtung nach einem der Ansprüche 1-10, wobei der Stützrahmen mehrere Verankerungen aufweist.

12. Baugruppe, die die Gefäßverschlussvorrichtung nach einem der Ansprüche 1-11 und ein langgestrecktes Abgabeelement aufweist, das zum Befestigen an der und zum Lösen von der Gefäßverschlussvorrichtung ausgebildet ist.

13. Baugruppe nach Anspruch 12, wobei das langgestreckte Abgabeelement und die Gefäßverschlussvorrichtung ineinandergreifende Arme auf weisen, wobei das langgestreckte Abgabeelement und die Gefäßverschlussvorrichtung ein Steckelement mit Außengewinde und ein Aufnahmeelement mit Innengewinde aufweisen oder wobei das langgestreckte Abgabeelement und die Gefäßverschlussvorrichtung zur Ablösung durch Elektrolyse ausgebildet sind.

14. Kit, das eine Gefäßverschlussvorrichtung nach einem der Ansprüche 1-11, eine rohrförmige Abgabevorrichtung und wahlweise ein langgestrecktes Abgabeelement aufweist, das zum Befestigen an der und zum Lösen von der Gefäßverschlussvorrichtung ausgebildet ist.

15. Kit nach Anspruch 14, wobei die Gefäßverschlussvorrichtung in der eingezwängten Form zusammengedrückt in der rohrförmigen Vorrichtung vorinstalliert ist.

## Revendications

1. Dispositif d'occlusion vasculaire (100) ayant une extrémité proximale (100p) et une extrémité distale (100d), ledit dispositif d'occlusion comprenant : (a) un bâti de support (110) comprenant un axe longitudinal (110x) et un centre longitudinal (110y), le bâti de support étant auto-expansible à partir d'une forme contrainte jusqu'à une forme non contrainte et comprenant (i) un raccord (112a), (ii) une pluralité de segments de fil radiaux (110a) s'étendant radialement à partir du raccord et (iii) une pluralité de segments de fil longitudinaux (110b) s'étendant longitudinalement le long du dispositif et (b) un insert sensiblement en forme de feuille (120) fixé sur ledit bâti de support, **caractérisé en ce que** l'insert sensiblement en forme de feuille (120) comprend (i) une première partie d'insert (120a) dans une première forme tridimensionnelle partiellement fermée qui comprend une première extrémité ouverte et une première extrémité fermée, dans lequel la première extrémité ouverte est orientée dans une direction proximale et la première extrémité fermée est positionnée à proximité du centre longitudinal et (ii) une seconde partie d'insertion (120b) dans une seconde forme tridimensionnelle partiellement fermée qui comprend une seconde extrémité ouverte et une seconde extrémité fermée, dans lequel la seconde extrémité ouverte est orientée dans une direction distale et la seconde extrémité fermée est positionnée à proximité du centre longitudinal (110y).

2. Dispositif d'occlusion vasculaire selon la revendication 1, dans lequel la première extrémité fermée de la partie d'insert est fixée sur la seconde extrémité fermée de la seconde partie d'insert.

3. Dispositif d'occlusion vasculaire selon l'une quelconque des revendications 1 à 2, dans lequel la première forme tridimensionnelle partiellement fermée et la seconde forme tridimensionnelle partiellement fermée sont sélectionnées parmi une forme conique creuse, une forme pyramidale creuse et une forme sphéroïdale partielle creuse.

4. Dispositif d'occlusion vasculaire selon l'une quelconque des revendications 1 à 3, dans lequel l'insert est un insert poreux, et/ou l'insert comprend un matériau polymère.

5. Dispositif d'occlusion vasculaire selon l'une quelconque des revendications 1 à 4, dans lequel le bâti de support comprend de quatre à huit segments de fil radiaux et de quatre à huit segments de fil longitudinaux.

6. Dispositif d'occlusion vasculaire selon l'une quelconque des revendications 1 à 5, dans lequel les segments de fil radiaux et les segments de fil longitudinaux sont linéaires ou sensiblement linéaires.

7. Dispositif d'occlusion vasculaire selon l'une quelconque des revendications 1 à 6, dans lequel lesdits segments de fil radiaux comprennent une première extrémité de segment de fil radial et une seconde extrémité de segment de fil radial, dans lequel la première extrémité de segment de fil radial est fixée au raccord, et dans lequel la seconde extrémité de segment de fil radial est fixée à l'un desdits segments de fil longitudinaux.

8. Dispositif d'occlusion vasculaire selon l'une quelconque des revendications 1 à 7, comprenant en outre un raccord supplémentaire et une pluralité de segments de fil radiaux supplémentaires s'étendant radialement à partir du raccord supplémentaire.

9. Dispositif d'occlusion vasculaire selon la revendication 8, dans lequel les segments de fil radiaux supplémentaires comprennent une première extrémité de segment de fil radial supplémentaire et une seconde extrémité de segment de fil radial supplémentaire, dans lequel la première extrémité de segment de fil radial supplémentaire est fixée au raccord supplémentaire, et dans lequel la seconde extrémité de segment de fil radial supplémentaire est fixée à l'un desdits segments de fil longitudinaux.

10. Dispositif d'occlusion vasculaire selon l'une quelconque des revendications 1 à 9, dans lequel le raccord comprend une caractéristique de fixation.

11. Dispositif d'occlusion vasculaire selon l'une quelconque des revendications 1 à 10, dans lequel le bâti de support comprend une pluralité d'ancrages.

12. Ensemble comprenant le dispositif d'occlusion vasculaire selon l'une quelconque des revendications 1 à 11 et un élément de pose allongé qui est configuré pour être fixé à et détaché du dispositif d'occlusion vasculaire.

13. Ensemble selon la revendication 12, dans lequel l'élément de pose allongé et le dispositif d'occlusion vasculaire comprend des bras de verrouillage, dans lequel l'élément de pose allongé et le dispositif d'occlusion vasculaire comprennent un élément mâle fileté et un réceptacle femelle fileté, ou bien dans lequel l'élément de pose allongé et le dispositif d'occlusion vasculaire sont configurés pour être détachés par électrolyse.

14. Kit comprenant un dispositif d'occlusion vasculaire selon l'une quelconque des revendications 1 à 11, un dispositif de pose tubulaire et facultativement un élément de pose allongé qui est configuré pour être fixé à et détaché du dispositif d'occlusion vasculaire.

15. Kit selon la revendication 14, dans lequel le dispositif d'occlusion vasculaire est comprimé et pré-chargé dans le dispositif tubulaire dans ladite forme contrainte.
